# EUROPEAN PATENT SPECIFICATION

(11) **EP 4 051 175 B1**
(45) Date of publication and mention of the grant of the patent: **09.10.2024**
(21) Application number: 20883262.6
(22) Date of filing: 21.10.2020
(51) Int. Cl.: A61F 2/16, A61F 9/00, A61L 27/34

(54) **SELF- VISCOELASTIC INTRAOCULAR LENS CARTRIDGE AND PRODUCTION METHOD THEREOF**
SELBSTVISKOELASTISCHE INTRAOKULARLINSENKARTUSCHE UND VERFAHREN ZU IHRER HERSTELLUNG
CARTOUCHE DE LENTILLE INTRAOCULAIRE AUTO-VISCOÉLASTIQUE ET SON PROCÉDÉ DE FABRICATION

(30) Priority: 01.11.2019 TR 201916931
(43) Date of publication of application: 07.09.2022
(73) Proprietor: VSY Biyoteknoloji Ve Ilac Sanayi Anonim Sirketi, Istanbul (TR)
(72) Inventor: ATMACA, Serkan, Tuzla/Istanbul (TR); OYTUN, Faruk, Tuzla/Istanbul (TR)
(74) Representative: Dericioglu, E. Korhan
(86) International application number: PCT/TR2020/050967
(87) International publication number: WO 2021/086289

(56) References cited:
- EP-A1- 2 241 286
- US-A1- 2003 096 131
- US-A1- 2010 125 279
- US-A1- 2010 256 651
- US-A1- 2010 256 651
- US-A1- 2013 253 402
- US-A1- 2014 316 425
- US-A1- 2016 287 438
- US-A1- 2018 200 047

## Description

### Field of the Invention

The present invention relates to a self-viscoelastic intraocular lens injector cartridge used for implanting the lens into the eye in cataract surgeries and production method thereof.

### Background of the Invention

Cartridge injector systems are used for implanting the lens used in cataract surgeries into the eye. The lens is folded in the cartridge and passed through the small diameter cartridge tunnel, and then unfolded in the lens capsule located in the eye. In the known state of the art, the cartridge body is produced from biocompatible materials (polypropylene, polycarbonate, polyethylene, polychlorotrifluoroethylene, polyvinyl chloride, polymethylmethacrylate, polystyrene) having suitable mechanical properties; however the surfaces of these materials are not suitable for the sliding of the cataract lens. In order for the lens to slide within the cartridge, at the 1st stage, either a lubricant additive is added to the material of the cartridge during the production process or the inner surface of the cartridge is coated with a lubricant film, and at the 2nd stage, during the application, a viscoelastic gel is added between the lens and the cartridge surface, allowing the lens to slide inside the cartridge.

The following problems can be seen in use of viscoelastic gels during insertion of the lens into the cartridge:
> Inability to adjust the amount of viscoelastic fluid used for sliding the lens in the injectors utilized in cataract surgery
> Inability to spread viscoelastic gel homogeneously,
> When low amount of viscoelastic gel is used, strains resulting during the passage of the lens and scratches and deformations occurring in the lens,
> When higher amount of viscoelastic gel is used, difficulty in cleaning after the implantation, the risk of changing the optical properties of the lens upon covering the surface of the lens,
> Viscoelastic being in different density types and use of the different density types of viscoelastic by the user,
> As the lens is pre-positioned in the cartridge in the new generation preloaded injectors, due to intense viscosity of the viscoelastic gel added later in these systems, inability of the viscoelastic gel to penetrate into the points at which the cartridge and the lens contact,
> The risk of the viscoelastic cannula contacting the lens and scratching the lens when viscoelastic is being applied in certain types of cartridges,
> Additional costs brought by the use of viscoelastic.

A device according to the preamble of claim 1 is known from the document US-A-2010/256651.

### Summary of the Invention

An object of the invention is to produce a self-viscoelastic intraocular lens injector cartridge that eliminates the need for external use of viscoelastic gel in cataract surgery.

Another object of the invention is to obtain a self-viscoelastic injector cartridge that can remain dry in the cartridge body until the step of intraocular injection of the lens in cataract surgery.

Another object of the invention is to obtain a thin viscoelastic film with biocompatible and biodegradable natural polymer content that can be activated by being easily hydrated upon application of a brine solution such as saline and balanced salt solution (bss) on the self-viscoelastic cartridge surface.

Another object of the invention is to avoid the effort to apply a lubricating viscoelastic gel to the cartridge before the operation and to further facilitate the process by using brine solutions such as saline and bss, by means of the viscoelastic film inherently provided in the cartridge body.

### Detailed Description of the Invention

**"Self-viscoelastic intraocular lens cartridge"** developed to fulfill the object of the present invention is illustrated in the accompanying figures, in which:

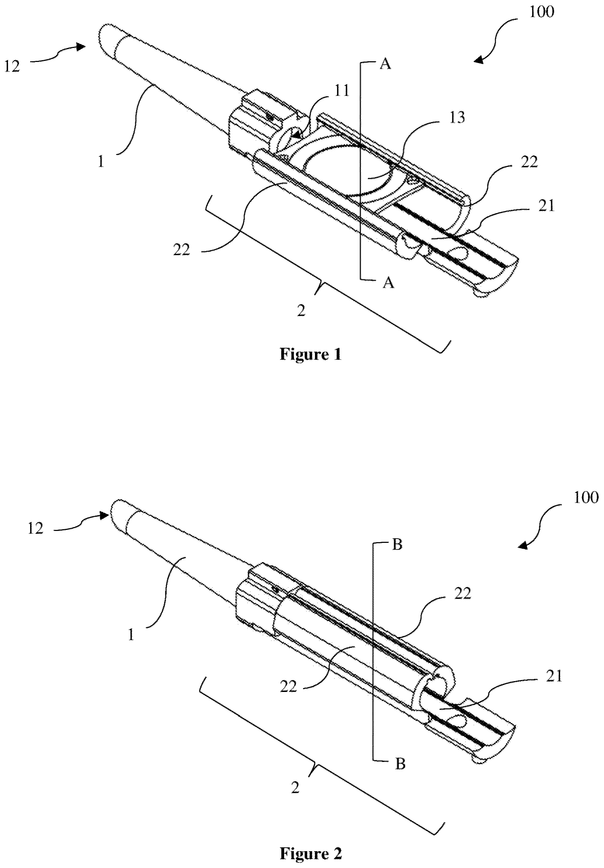

- **Figure 1 -**: is a perspective view of the open state of the viscoelastic intraocular lens cartridge according to the invention.
- **Figure 2** -: is a perspective view of the closed state of the viscoelastic intraocular lens cartridge according to the invention.

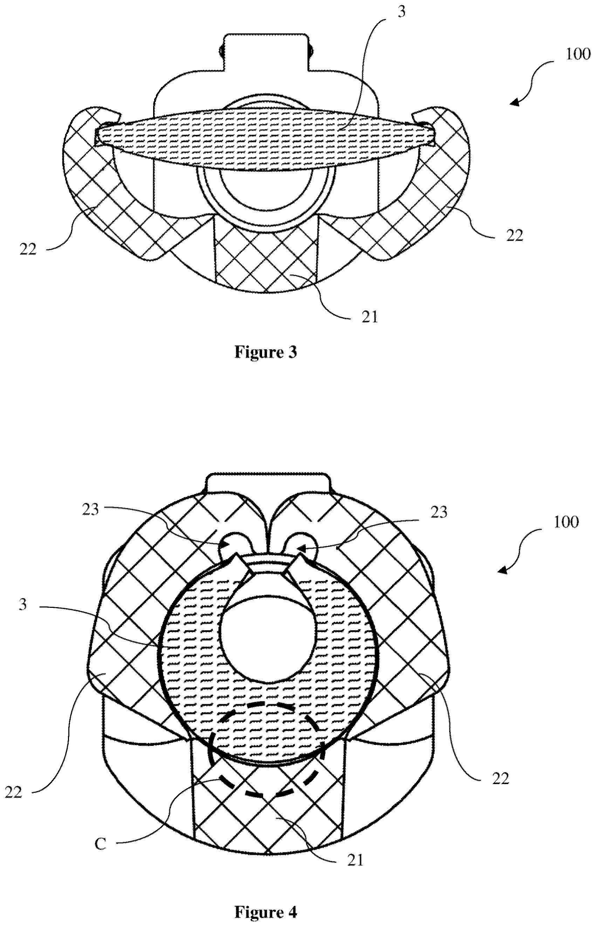

- **Figure 3** -: is a view of the A-A section shown in Figure 1 of the viscoelastic intraocular lens cartridge according to the invention.
- **Figure 4** -: is the view of the B-B section shown in Figure 2 of the viscoelastic intraocular lens cartridge according to the invention.

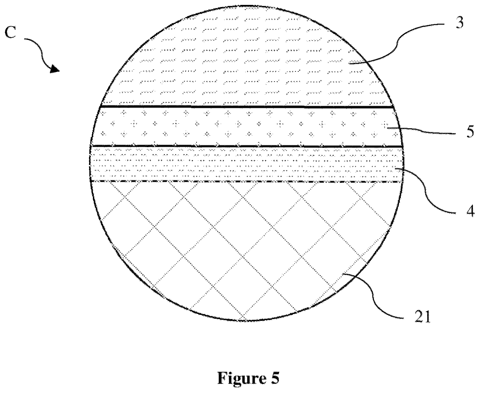

- **Figure 5** -: is a magnified view of the C portion in Figure 4.

The components shown in the figures are each given reference numbers as follows:
**100.** Self-viscoelastic intraocular lens cartridge
**1.** Cartridge end
   **11.** Feed orifice
   **12.** Outlet orifice
**2.** Cartridge body
   **21.** Base
   **22.** Gripping arm
   **23.** Slot
**3.** Cataract lens
**4.** Coating layer
**5.** Viscoelastic film

Self-viscoelastic intraocular lens cartridge (100) of the present invention for use in implanting a lens into an eye in cataract surgeries, comprises:
- at least one cartridge end (1) including a tunnel extending longitudinally between a feed orifice (11) and an outlet orifice (12),
- at least one cartridge body (2) coupled to the feed orifice (11) side of the cartridge end (1), having:
   o at least one base (21) having a long rectangular shape, one short side of which is integrated with the feed orifice (11); and
   o at least two gripping arms (22) which extend along the long sides of the said base (21) and are coupled to the said sides such that they can rotate about the axis of the said sides and which thus enables to create a hollow cylindrical form when in the closed state in which the long sides that are not coupled are brought closer to each another,
   o at least one slot (23) in each gripping arm (22), which is located on the long sides of the gripping arms (22) that are not coupled to the base (21), and which allows a cataract lens (3) that is positioned by being folded into the cylindrical gap within the hollow cylindrical structure formed in the closed state to be held by the edges thereof in the open position in which the unfixed sides of the gripping arms (22) move away from each other,
- at least one coating layer (4), which is applied to the entire lateral surface of the cylindrical gap inside the cartridge body (2), and which is thus provided on the surface of the gripping arms (22) and the base (21) facing the cylindrical gap,
- a viscoelastic film (5); which is a rigid film layer comprising at least one biocompatible and biodegradable natural polymer applied on the coating layer (4) and provided between the coating layer and the cataract lens (3), which, when the cartridge body (2) is in closed state, is folded in the inner gap to acquire a form in accordance with the lateral surface of the inner gap; and which, when the user applies saline or bss (brine solution) to the cartridge body (2), is activated and becomes a lubricious gel to enable the cataract lens (3) to be driven through the inner gap of the cartridge body (2) towards the feed orifice (11) of the cartridge end (1) and from the feed orifice (11) to the outlet orifice (12) without being damaged.

Within the scope of the invention, a viscoelastic film (5) with a homogenous thickness is obtained on the inner surface of the cartridge by coating the inner surface thereof with a viscoelastic gel during production process of the self-viscoelastic intraocular lens cartridge (100). The viscoelastic film (5) on the surface of the inner gap of the cartridge body (2) adheres tightly to the cartridge material and hardens as a thin film in that area. The viscoelastic intraocular lens cartridge (100) of the present invention provides an advantage to the end user in that it is provided as a self-viscoelastic film (5). Before the operation, the user applies saline or bss (brine solution) to the cartridge body (2) whereby enabling the dry viscoelastic film (5) to be activated and acquire a lubricous gel consistency. With the lubricious surface formed, the cataract lens (3) easily slides through the cartridge body (2) and the cartridge end (1), and thereby passes through the tunnel, and exits through the outlet orifice (12) of the cartridge end (1) without being deformed or having difficulty.

The cataract lens (3) is implanted into the eye by being folded. Cartridge injector systems are used for the implantation of the cataract lens (3) into the eye. The cartridge body (2) is made of a biocompatible material and is used for folding the cataract lens (3) and delivering it through the conical channel of the cartridge end (1) to the human eye. In the present invention, the cartridge body (2) is made of a biocompatible polypropylene material with suitable mechanical properties. The surface of the polypropylene material is not suitable for the sliding of the cataract lens (3). In order to add lubricity to the surface of this material, in conventional methods, the cartridge is first coated with polymer-based coating materials at the production phase, then a viscoelastic gel is applied during the operation to provide the necessary lubricity for the lens to pass through the cartridge. Within the scope of the present invention, a cartridge with a self-viscoelastic lubricious surface is obtained by coating the inner gap surface of the cartridge body (2) with a viscoelastic gel in the form of a thin film during production.

Within the scope of the present invention, the method of producing a self-viscoelastic intraocular lens cartridge (100) comprises the following steps:
- Producing the cartridge end (1) and the cartridge body (2) by plastic injection method,
- Subsequently, applying plasma treatment to the surfaces of the cartridge end (1) and the cartridge body (2) to make the surfaces ready for the chemical coating
- Then, applying pre-coating material to the inner surface of the cartridge,
- Subsequently, enabling the coating material, which is cured by applying heat treatment, to be bonded with the inner surfaces of the cartridge end (1) and the cartridge body (2),
- After the coating process, applying the viscoelastic gel containing biocompatible and biodegradable natural polymer onto the inner surface of the cartridge,
- Drying the viscoelastic film (5) by heat treatment.

The viscoelastic film (5) of the viscoelastic intraocular lens cartridge (100) of the present invention remains dry until the application. During the application, the surface of the cartridge body (2) is activated by being hydrated with a brine solution such as saline and bss. Activation takes place in a short period of time such as 3-5 seconds and a wet lubricious form is obtained on the surface. During implantation, the cataract lens (3) is safely implanted into the eye by sliding through this lubricious viscoelastic film (5) layer. The viscoelastic intraocular lens cartridge (100) according to the present invention can be used in all injection, implantation and injector systems where viscoelastic gel can be used as a lubricant.

## Claims

1. Self-viscoelastic intraocular lens cartridge (100) for use in implanting a lens into an eye in cataract surgeries and comprising:
- at least one cartridge end (1) including a tunnel extending longitudinally between a feed orifice (11) and an outlet orifice (12),
- at least one cartridge body (2) coupled to the feed orifice (11) side of the cartridge end (1), having:
o at least one base (21) having a long rectangular shape, one short side of which is integrated with the feed orifice (11); and
o at least two gripping arms (22) which extend along the long sides of the said base (21) and are coupled to the said sides such that they can rotate about the axis of the said sides and which thus enables to create a hollow cylindrical form when in the closed state in which the long sides that are not coupled are brought closer to each another,
o at least one slot (23) in each gripping arm (22), which is located on the long sides of the gripping arms (22) that are not coupled to the base (21), and which allows a cataract lens (3) that is positioned by being folded into the cylindrical gap within the hollow cylindrical structure formed in the closed state to be held by the edges thereof in the open position in which the unfixed sides of the gripping arms (22) move away from each other,
- at least one coating layer (4), which is applied to the entire lateral surface of the cylindrical gap inside the cartridge body (2), and which is thus provided on the surface of the gripping arms (22) and the base (21) facing the cylindrical gap; and **characterized by**
- a viscoelastic film (5); which is a dry film layer comprising at least one biocompatible and biodegradable natural polymer applied on the coating layer (4) and provided between the coating layer and the cataract lens (3), which, when the cartridge body (2) is in closed state, is folded in the inner gap to acquire a form in accordance with the lateral surface of the inner gap; and which, when the user applies saline or bss (brine solution) to the cartridge body (2), is activated and becomes a lubricious gel to enable the cataract lens (3) to be driven through the inner gap of the cartridge body (2) towards the feed orifice (11) of the cartridge end (1) and from the feed orifice (11) to the outlet orifice (12) without being damaged.

2. Self-viscoelastic intraocular lens cartridge (100) according to claim 1 **characterized by** the
cartridge body (2) which is produced from polypropylene material in order for it to be biocompatible and have suitable mechanical properties.

3. Method of producing a self-viscoelastic intraocular lens cartridge (100) according to Claim 1, **characterized in that** it comprises the following steps:
- Producing the cartridge end (1) and the cartridge body (2) by plastic injection method,
- Subsequently, applying plasma treatment to the surfaces of the cartridge end (1) and the cartridge body (2) to make the surfaces ready for the chemical coating,
- Then, applying coating material to the inner surface of the cartridge,
- Removing the waste materials from the surface by centrifugation method,
- Subsequently, enabling the coating material, which is cured in a drying oven, to be bonded with the surfaces of the cartridge end (1) and the cartridge body (2),
- After the coating process, applying the viscoelastic gel onto the inner surface of the cartridge as a thin film,
- Drying the viscoelastic film (5) by heat treatment.

## Patentansprüche

1. Selbstviskoelastische Intraokularlinsenpatrone (100) zur Verwendung beim Implantieren einer Linse in ein Auge bei Kataraktoperationen, umfassend:
- mindestens ein Patronenende (1) mit einem Tunnel, der sich in Längsrichtung zwischen einer Zuführöffnung (11) und einer Auslassöffnung (12) erstreckt,
- mindestens einen Patronenkörper (2), der an der Seite der Zuführöffnung (11) mit dem Patronenende (1) gekoppelt ist, aufweisend:
∘ mindestens einen Sockel (21) mit einer länglichen rechteckigen Form, dessen eine kurze Seite in die Zuführöffnung (11) integriert ist; und
o mindestens zwei Greifarme (22), die sich entlang der Längsseiten des Sockels (21) erstrecken und mit den genannten Seiten so verbunden sind, dass sie sich um die Achse der Seiten drehen können, wodurch im geschlossenen Zustand eine hohlzylindrische Form erzeugt werden kann, bei der die nicht verbundenen Längsseiten einander angenähert werden,
∘ mindestens einen Schlitz (23) in jedem Greifarm (22), der sich an den Längsseiten der Greifarme (22) befindet, die nicht mit dem Sockel (21) gekoppelt sind, und der es ermöglicht, dass eine Kataraktlinse (3), die durch Falten in den zylindrischen Spalt innerhalb der hohlen zylindrischen Struktur, die im geschlossenen Zustand gebildet wird, positioniert wird, von den Rändern derselben in der offenen Position gehalten wird, in der sich die nicht fixierten Seiten der Greifarme (22) voneinander weg bewegen,
- mindestens eine Überzugssschicht (4), die auf der gesamten Seitenfläche des zylindrischen Spalts im Inneren des Patronenkörpers (2) aufgebracht ist und die auf der dem zylindrischen Spalt zugewandten Oberfläche der Greifarme (22) und des Sockels (21) vorgesehen ist; und **gekennzeichnet durch,**
- einen viskoelastischen Film (5), bei dem es sich um eine trockene Folienschicht handelt, die mindestens ein biokompatibles und biologisch abbaubares natürliches Polymer umfasst, das auf die Überzugsschicht (4) aufgebracht und zwischen der Überzugsschicht und der Kataraktlinse (3) vorgesehen ist, der, wenn sich der Patronenkörper (2) in geschlossenem Zustand befindet, im inneren Spalt gefaltet wird, um eine Form entsprechend der Seitenfläche des inneren Spalts anzunehmen; und der, wenn der Benutzer Kochsalzlösung oder bss (Salzlösung) auf den Patronenkörper (2) aufträgt, aktiviert wird und zu einem Gleitgel wird, damit die Kataraktlinse (3) durch den inneren Spalt des Patronenkörpers (2) zur Zufühsöffnung (11) des Patronenendes (1) und von der Zuführöffnung (11) zur Auslassöffnung (12) getrieben werden kann, ohne beschädigt zu werden.

2. Selbstviskoelastische Intraokularlinsenpatrone (100) nach Anspruch 1, **gekennzeichnet durch** den Patronenkörper (2), der aus Polypropylenmaterial hergestellt ist, sodass er biokompatibel ist und geeignete mechanische Eigenschaften aufweist.

3. Verfahren zur Herstellung einer selbstviskoelastischen Intraokularlinsenpatrone (100) nach Anspruch 1, **dadurch gekennzeichnet, dass** es die folgenden Schritte umfasst:
- Herstellung des Patronenendes (1) und des Patronenkörpers (2) durch Kunststoff-Spritzgussverfahren,
- Anschließende Plasmabehandlung der Oberflächen des Patronenendes (1) und des Patronenkörpers (2), um die Oberflächen für die chemische Beschichtung vorzubereiten,
- Auftragen des Beschichtungsmaterials auf die Innenfläche der Patrone,
- Entfernen der Abfallstoffe von der Oberfläche durch Zentrifugieren,
- Anschließende Kontaktierung des Beschichtungsmaterials, das in einem Trockenofen ausgehärtet wird, mit den Oberflächen des Patronenendes (1) und des Patronenkörpers (2),
- Aufbringen des viskoelastischen Gels auf die Innenfläche der Patrone als dünne Folie im Anschluss an den Beschichtungsvorgang,
- Trocknen der viskoelastischen Folie (5) durch Wärmebehandlung.

## Revendications

1. Cartouche de lentille intraoculaire auto-viscoélastique (100) utilisée pour implanter une lentille dans un oeil lors d'opérations de la cataracte et comprenant
- au moins une extrémité de cartouche (1) comprenant un tunnel s'étendant longitudinalement entre un orifice d'alimentation (11) et un orifice de sortie (12);
- au moins un corps de cartouche (2) couplé à l'orifice d'alimentation (11) du côté de l'extrémité de la cartouche (1), ayant :
∘ au moins une base (21) de forme rectangulaire longue, dont un petit côté est intégré à l'orifice d'alimentation (11) ; et
∘ au moins deux bras de préhension (22) qui s'étendent le long des côtés longs de ladite base (21) et sont couplés auxdits côtés de manière à pouvoir tourner autour de l'axe desdits côtés et qui permet ainsi de créer une forme cylindrique creuse à l'état fermé dans laquelle les côtés longs qui ne sont pas couplés sont rapprochés l'un de l'autre,
∘ au moins une fente (23) dans chaque bras de préhension (22), qui est située sur les côtés longs des bras de préhension (22) qui ne sont pas couplés à la base (21), et qui permet à une lentille de cataracte (3) qui est positionnée en étant pliée dans l'espace cylindrique à l'intérieur de la structure cylindrique creuse formée à l'état fermé d'être maintenue par ses bords dans la position ouverte dans laquelle les côtés dépliés des bras de préhension (22) s'éloignent l'un de l'autre.
- au moins une couche de revêtement (4), qui est appliquée sur toute la surface latérale de l'espace cylindrique à l'intérieur du corps de la cartouche (2), et qui se trouve donc sur la surface des bras de préhension (22) et de la base (21) faisant face à l'espace cylindrique et **caractérisée par** :
- un film viscoélastique (5), qui est une couche de film sec comprenant au moins un polymère naturel biocompatible et biodégradable appliqué sur la couche de revêtement (4) et placé entre la couche de revêtement et la lentille de la cataracte (3), qui, lorsque le corps de la cartouche (2) est fermé, est plié dans l'espace intérieur pour acquérir une forme conforme à la surface latérale de l'espace intérieur; et qui, lorsque l'utilisateur applique une solution saline ou bss (solution saline) sur le corps de la cartouche (2), est activé et devient un gel lubrifiant pour permettre à la lentille de la cataracte (3) d'être conduite à travers l'espace intérieur du corps de la cartouche (2) vers l'orifice d'alimentation (11) de l'extrémité de la cartouche (1) et de l'orifice d'alimentation (11) à l'orifice de sortie (12) sans être endommagée.

2. Cartouche de lentille intraoculaire auto-viscoélastique (100) selon la revendication 1, **caractérisée par** le corps de la cartouche (2) qui est fabriqué en polypropylène afin d'être biocompatible et d'avoir des propriétés mécaniques appropriées.

3. Procédé de fabrication d'une cartouche de lentille intraoculaire auto-viscoélastique (100) selon la revendication 1, **caractérisé en ce qu'**il comprend les étapes suivantes :
- Production de l'extrémité de la cartouche (1) et du corps de la cartouche (2) par une méthode d'injection plastique ;
- Ensuite, application d'un traitement au plasma sur les surfaces de l'extrémité de la cartouche (1) et du corps de la cartouche (2) pour préparer les surfaces au revêtement chimique ;
- Puis, application d'un matériau de revêtement sur la surface intérieure de la cartouche ;
- Élimination des déchets de la surface par centrifugation ;
- Ensuite, le matériau de revêtement, qui est durci dans un four de séchage, peut être lié aux surfaces de l'extrémité de la cartouche (1) et du corps de la cartouche (2),
- Après le processus revêtement, le gel viscoélastique est appliqué sur la surface intérieure de la cartouche sous la forme d'un film mince ;
- Séchage du film viscoélastique (5) par traitement thermique.
